# EUROPEAN PATENT APPLICATION

(11) **EP 4 670 624 A1**
(43) Date of publication of application: **31.12.2025**
(21) Application number: 24777412.8
(22) Date of filing: 08.01.2024
(51) Int. Cl.: A61B 5/0225, A61B 5/022, A61B 5/00, A61B 5/318

(54) **BLOOD PRESSURE MEASUREMENT METHOD AND WEARABLE DEVICE**

(30) Priority: 31.03.2023 CN 202310386736
(71) Applicant: Huawei Technologies Co., Ltd., Shenzhen, Guangdong 518129 (CN)
(72) Inventor: ZHANG, Hui, Shenzhen, Guangdong 518129 (CN); JIA, Zheng, Shenzhen, Guangdong 518129 (CN); ZHU, Yu, Shenzhen, Guangdong 518129 (CN)
(74) Representative: Maiwald GmbH
(86) International application number: PCT/CN2024/071204
(87) International publication number: WO 2024/198616

(57) **Abstract**

A blood pressure measurement method and a wearable device are disclosed, and relate to the field of terminal technologies, to capture blood pressure in a peak time period, resolve a problem that it is difficult to discover morning-surge hypertension, masked hypertension, and the like, and provide more comfortable blood pressure measurement experience to a user. The method is applied to the wearable device, and includes: starting a first manner to measure user blood pressure in a first time period; determining a time period of abnormal blood pressure based on the user blood pressure and a measurement time period corresponding to the user blood pressure; and starting a second manner to measure user blood pressure in the time period of abnormal blood pressure, where the second manner is different from the first manner.

## Description

This application claims priority to Chinese Patent Application No. 202310386736.6, filed with the China National Intellectual Property Administration on March 31, 2023 and entitled "BLOOD PRESSURE MEASUREMENT METHOD AND WEARABLE DEVICE", which is incorporated herein by reference in its entirety.

### TECHNICAL FIELD

This application relates to the field of terminal technologies, and in particular, to a blood pressure measurement method and a wearable device.

### BACKGROUND

Hypertension, as a serious chronic disease, increases risks of developing heart diseases, brain diseases, kidney diseases, and other diseases, and is a risk factor for cardiovascular and cerebrovascular diseases. Therefore, effective measurement of blood pressure is crucial to increasing an awareness rate, a control rate, and the like of hypertension.

However, human blood pressure is constantly changing. It is best to measure blood pressure in a time period in which blood pressure peaks. In this way, if blood pressure in the peak time period satisfies a standard, it can be determined that user blood pressure satisfies the standard. Therefore, how to capture blood pressure in a peak time period is crucial.

### SUMMARY

This application provides a blood pressure measurement method and a wearable device, to capture blood pressure in a peak time period, resolve a problem that it is difficult to discover morning-surge hypertension, masked hypertension, and the like, and provide more comfortable blood pressure measurement experience to a user.

To achieve the foregoing objectives, this application uses the following technical solutions.

According to a first aspect, a blood pressure measurement method is provided and applied to a wearable device. The method includes: starting a first manner to measure user blood pressure in a first time period; determining a time period of abnormal blood pressure based on the user blood pressure and a measurement time period corresponding to the user blood pressure; and starting a second manner to measure user blood pressure in the time period of abnormal blood pressure, where the second manner is different from the first manner.

According to the foregoing technical solution, the wearable device may continuously monitor user blood pressure in the first manner, determine a time period of abnormal blood pressure based on the monitored user blood pressure and a monitoring time, and further measure user blood pressure in the time period of abnormal blood pressure in the second manner different from the first manner. For example, the first manner may be a manner in which a user has no perception, and the second manner may be a manner in which the user has perception. In this case, frequent measurement of user blood pressure in the manner in which the user has no perception does not cause discomfort to the user, and can provide more comfortable blood pressure measurement experience to the user. Further, the time period of abnormal blood pressure may be determined based on the monitored blood pressure and the monitoring time. The time period of abnormal blood pressure may be a peak time period of blood pressure. Then, user blood pressure in the time period of abnormal blood pressure is measured in a perceptible manner. In this way, blood pressure in the peak time period can be more accurately captured, to resolve a problem that it is difficult to discover morning-surge hypertension, masked hypertension, and the like.

In a possible design, the first manner includes a manner of determining user blood pressure based on at least one of a photoplethysmography PGG signal, a pressure sensor signal, and an electrocardiogram. The second manner includes a manner of determining user blood pressure according to oscillography. According to this design, the user has no perception in the use of the manner of determining user blood pressure based on at least one of a photoplethysmography PGG signal, a pressure sensor signal, and an electrocardiogram, and the user has perception in the use of the manner of determining user blood pressure according to oscillography. Therefore, obtaining the time period of abnormal blood pressure by continuously monitoring user blood pressure in the manner in which the user has no perception does not cause discomfort to the user, and can provide more comfortable blood pressure measurement experience to the user. Measurement in the manner in which the user has perception is more accurate than measurement in the manner in which the user has no perception. Therefore, further monitoring blood pressure in the time period of abnormal blood pressure in the manner in which the user has perception can more accurately capture blood pressure in a peak time period, and resolve a problem that it is difficult to discover morning-surge hypertension, masked hypertension, and the like.

In a possible design, determining the time period of abnormal blood pressure based on the user blood pressure and the measurement time period corresponding to the user blood pressure includes: if user blood pressure measured in a time period included in the first time period satisfies a preset blood pressure abnormality condition, determining that the time period is the time period of abnormal blood pressure. According to this design, when it is determined that user blood pressure measured in a time period satisfies the preset blood pressure abnormality condition, it may be determined that the time period is the time period of abnormal blood pressure. Further, user blood pressure in the time period of abnormal blood pressure is measured in the manner in which the user has perception. In this way, blood pressure in a peak time period can be more accurately captured, to resolve a problem that it is difficult to discover morning-surge hypertension, masked hypertension, and the like.

In a possible design, the preset blood pressure abnormality condition includes at least one of a blood pressure condition, a fluctuation condition, and a quantity condition. The blood pressure condition includes: user blood pressure is not within a preset blood pressure range, and/or a user blood pressure curve does not match a preset curve. The fluctuation condition includes a first fluctuation condition, and the first fluctuation condition includes: a fluctuation value of user blood pressure is not within a preset fluctuation range. The quantity condition includes: for a time period, a proportion of a quantity of measured user blood pressure satisfying the blood pressure condition and/or the fluctuation condition to a quantity of time periods included in the first time period is within a first proportion range, or the quantity of measured user blood pressure satisfying the blood pressure condition and/or the fluctuation condition is within a first quantity range. According to this design, it may be determined, based on the blood pressure condition, the fluctuation condition, the quantity condition, or the like, whether a time period is the time period of abnormal blood pressure. Further, user blood pressure in the time period of abnormal blood pressure is measured in the manner in which the user has perception. In this way, blood pressure in a peak time period can be more accurately captured, to resolve a problem that it is difficult to discover morning-surge hypertension, masked hypertension, and the like.

In a possible design, the time period of abnormal blood pressure is a daytime time period. Starting the second manner to measure the user blood pressure in the time period of abnormal blood pressure includes: starting the second manner to measure the user blood pressure in the time period of abnormal blood pressure at high frequency. According to this design, because the user blood pressure is measured in a daytime scene and the user is in an awake state, when the user blood pressure is measured in the manner in which the user has perception, the measurement at the high frequency causes weak discomfort to the user. In this way, a physical status of the user is not affected, and a more accurate blood pressure situation of the user can be obtained.

In a possible design, the first time period includes n daytimes, and n is greater than 0. After determining the time period of abnormal blood pressure based on the user blood pressure and the measurement time period corresponding to the user blood pressure, the method further includes: if in the first time period, a quantity of daytimes among which a proportion of a total length of a time period of abnormal blood pressure included in a daytime to a total length of the daytime is within a second proportion range is greater than a second quantity range, or a quantity of daytimes among which a total length of a time period of abnormal blood pressure included in a daytime is within a first length range is greater than the second quantity range, starting the second manner to measure user blood pressure in the daytimes. According to this design, when a time period of abnormal blood pressure included in a daytime satisfies the foregoing condition, it indicates that the user often has blood pressure abnormalities throughout the daytime. In other words, the user may have a hypertension risk throughout the daytime. Measuring user blood pressure throughout a daytime in the manner in which the user has perception can obtain a more accurate blood pressure situation of the user.

In a possible design, the fluctuation condition further includes a second fluctuation condition and a third fluctuation condition. The second fluctuation condition includes: user blood pressure is greater than or equal to a first fluctuation threshold and is less than or equal to a second fluctuation threshold. The third fluctuation condition includes: user blood pressure is greater than the second fluctuation threshold.

In a possible design, the time period of abnormal blood pressure is at night. Starting the second manner to measure the user blood pressure in the time period of abnormal blood pressure includes: if the second fluctuation condition is satisfied in the time period of abnormal blood pressure, starting the second manner to measure the user blood pressure in the time period of abnormal blood pressure at low frequency. According to this design, when the second fluctuation condition is satisfied in the time period of abnormal blood pressure, it indicates that fluctuation of blood pressure in the time period of abnormal blood pressure is small. When measuring the user blood pressure in the time period of abnormal blood pressure in the manner in which the user has perception, the wearable device may measure the user blood pressure at the low frequency. In this case, real blood pressure of the user can be reflected when blood pressure measurement is performed a small quantity of times, so that power consumption of the wearable device can be reduced when blood pressure in a peak time period is accurately captured.

In a possible design, the time period of abnormal blood pressure is at night. Starting the second manner to measure the user blood pressure in the time period of abnormal blood pressure includes: if the third fluctuation condition is satisfied in the time period of abnormal blood pressure, starting the second manner to measure the user blood pressure in the time period of abnormal blood pressure at high frequency. According to this design, when the third fluctuation condition is satisfied in the time period of blood pressure fluctuation, it indicates that fluctuation in the time period of abnormal blood pressure is large. When measuring the user blood pressure in the time period of abnormal blood pressure in the perceptible manner, the wearable device may measure the user blood pressure at the high frequency. In this case, real blood pressure of the user can be reflected only when blood pressure measurement is performed a large quantity of times. In this way, blood pressure in a peak time period can be accurately captured.

In a possible design, before starting the second manner to measure the user blood pressure in the time period of abnormal blood pressure, the method further includes: outputting a reminder message, where the reminder message is used to remind the user to start, in the time period of abnormal blood pressure, the second manner to measure the user blood pressure. According to this design, the user may start, according to an actual requirement, the perceptible manner to measure the blood pressure in the time period of abnormal blood pressure, so that an actual blood pressure measurement requirement of the user can be satisfied, and a probability of causing uncomfortable blood pressure measurement experience to the user is reduced.

In a possible design, before outputting the reminder message, the method further includes: determining that the time period of abnormal blood pressure is a daytime time period. According to this design, in a daytime scene, the wearable device outputs the reminder message, to remind the user to start, in the time period of abnormal blood pressure, the perceptible manner to measure the user blood pressure in the time period of abnormal blood pressure. In the daytime scene, a probability that the user is in a sleep state is low, so that a probability of disturbing the user can be reduced. In addition, a probability that the user sees the reminder message is high, so that the perceptible manner can be started in the time period of abnormal blood pressure in time to measure user blood pressure, to obtain a more accurate blood pressure situation of the user.

In a possible design, starting the second manner to measure the user blood pressure in the time period of abnormal blood pressure includes: receiving a user operation; and in response to the user operation, starting the second manner to measure the user blood pressure in the time period of abnormal blood pressure. According to this design, in response to the user operation, the second manner is started to measure the user blood pressure in the time period of abnormal blood pressure. Because the second manner is a blood pressure measurement manner in which the user has perception, an actual requirement of the user can be satisfied, and a probability of causing uncomfortable blood pressure measurement experience to the user is reduced.

According to a second aspect, a wearable device is provided. The wearable device has a function of implementing the method according to the first aspect and any one of the designs of the first aspect. The function may be implemented by hardware, or may be implemented by hardware executing corresponding software. The hardware or the software includes one or more modules corresponding to the function. In a possible example, the wearable device includes a processing unit (or referred to as a processing module). The processing unit is configured to: start a first manner to measure user blood pressure in a first time period; determine a time period of abnormal blood pressure based on the user blood pressure and a measurement time period corresponding to the user blood pressure; and start a second manner to measure user blood pressure in the time period of abnormal blood pressure, where the second manner is different from the first manner.

In a possible design, the first manner includes a manner of determining user blood pressure based on at least one of a photoplethysmography PGG signal, a pressure sensor signal, and an electrocardiogram. The second manner includes a manner of determining user blood pressure according to oscillography.

In a possible design, the processing unit is specifically configured to: if user blood pressure measured in a time period included in the first time period satisfies a preset blood pressure abnormality condition, determine that the time period is the time period of abnormal blood pressure.

In a possible design, the preset blood pressure abnormality condition includes at least one of a blood pressure condition, a fluctuation condition, and a quantity condition. The blood pressure condition includes: user blood pressure is not within a preset blood pressure range, and/or a user blood pressure curve does not match a preset curve. The fluctuation condition includes a first fluctuation condition, and the first fluctuation condition includes: a fluctuation value of user blood pressure is not within a preset fluctuation range. The quantity condition includes: for a time period, a proportion of a quantity of measured user blood pressure satisfying the blood pressure condition and/or the fluctuation condition to a quantity of time periods included in the first time period is within a first proportion range, or the quantity of measured user blood pressure satisfying the blood pressure condition and/or the fluctuation condition is within a first quantity range.

In a possible design, the time period of abnormal blood pressure is a daytime time period. The processing unit is specifically configured to start the second manner to measure the user blood pressure in the time period of abnormal blood pressure at high frequency.

In a possible design, the first time period includes n daytimes, and n is greater than 0. The processing unit is further configured to: if in the first time period, a quantity of daytimes among which a proportion of a total length of a time period of abnormal blood pressure included in a daytime to a total length of the daytime is within a second proportion range is greater than a second quantity range, or a quantity of daytimes among which a total length of a time period of abnormal blood pressure included in a daytime is within a first length range is greater than the second quantity range, start the second manner to measure user blood pressure in the daytimes.

In a possible design, the fluctuation condition further includes a second fluctuation condition and a third fluctuation condition. The second fluctuation condition includes: user blood pressure is greater than or equal to a first fluctuation threshold and is less than or equal to a second fluctuation threshold. The third fluctuation condition includes: user blood pressure is greater than the second fluctuation threshold.

In a possible design, the time period of abnormal blood pressure is at night. The processing unit is specifically configured to: if the second fluctuation condition is satisfied in the time period of abnormal blood pressure, start the second manner to measure the user blood pressure in the time period of abnormal blood pressure at low frequency.

In a possible design, the time period of abnormal blood pressure is at night. The processing unit is specifically configured to: if the third fluctuation condition is satisfied in the time period of abnormal blood pressure, start the second manner to measure the user blood pressure in the time period of abnormal blood pressure at high frequency.

In a possible design, the processing unit is further configured to output a reminder message, where the reminder message is used to remind a user to start, in the time period of abnormal blood pressure, the second manner to measure the user blood pressure.

In a possible design, the processing unit is further configured to determine that the time period of abnormal blood pressure is a daytime time period.

In a possible design, the processing unit is specifically configured to: receive a user operation; and in response to the user operation, start the second manner to measure the user blood pressure in the time period of abnormal blood pressure.

According to a third aspect, a wearable device is provided, including a processor, a memory, and a sensor. The memory and the sensor are coupled to the processor, the memory is configured to store computer program code, the computer program code includes computer instructions, and the processor reads the computer instructions from the memory, to enable the wearable device to perform the method according to the first aspect and any one of the designs of the first aspect.

For example, the sensor includes one or more of a PPG sensor and a pressure sensor.

In a possible design, the wearable device may further include a display, and the display may be used by the electronic device to perform a display operation, for example, output a reminder message.

In a possible design, the wearable device further includes a communication interface, and the communication interface may be used by the wearable device to communicate with another apparatus (for example, an electronic device). For example, the communication interface may be a transceiver, an input/output interface, an interface circuit, an output circuit, an input circuit, a pin, a related circuit, or the like.

According to a fourth aspect, a computer-readable storage medium is provided. The computer-readable storage medium includes a computer program or instructions. When the computer program or the instructions are run on a wearable device, the wearable device is enabled to perform the method according to the first aspect and any one of the designs of the first aspect.

According to a fifth aspect, a computer program product is provided. When the computer program product is run on a computer, the computer is enabled to perform the method according to the first aspect and any one of the designs of the first aspect.

According to a sixth aspect, a circuit system is provided. The circuit system includes a processing circuit, and the processing circuit is configured to perform the method according to the first aspect and any one of the designs of the first aspect.

According to a seventh aspect, a chip system is provided, including at least one processor and at least one interface circuit. The at least one interface circuit is configured to: perform a transceiver function, and send instructions to the at least one processor. When the at least one processor executes the instructions, the at least one processor performs the method according to the first aspect and any one of the designs of the first aspect.

It should be noted that, for technical effects achieved by any one of the designs of the second aspect to the seventh aspect, refer to technical effects achieved by a corresponding design of the first aspect. Details are not described herein again.

### BRIEF DESCRIPTION OF DRAWINGS

FIG. 1 is a diagram of a structure of a wearable device according to an embodiment of this application;
FIG. 2 is a diagram of a structure of another wearable device according to an embodiment of this application;
FIG. 3 is a diagram of a blood pressure curve according to an embodiment of this application;
FIG. 4 to FIG. 10(1) to FIG. 10(3) are respectively interface diagrams 1 to 7 according to an embodiment of this application;
FIG. 11 is a diagram of a structure of another wearable device according to an embodiment of this application; and
FIG. 12 is a diagram of a structure of a chip system according to an embodiment of this application.

### DESCRIPTION OF EMBODIMENTS

The following describes technical solutions in embodiments of this application in detail with reference to accompanying drawings.

The terms "include", "have", and any other variant thereof mentioned in descriptions of this application are intended to cover a non-exclusive inclusion. For example, a process, a method, a system, a product, or a device that includes a series of steps or units is not limited to listed steps or units, but optionally further includes other unlisted steps or units, or optionally further includes another inherent step or unit of the process, the method, the product, or the device.

It should be noted that, in embodiments of this application, an expression such as "example" or "for example" is used to represent giving an example, an illustration, or a description. Any embodiment or design scheme described as an "example" or "for example" in embodiments of this application shall not be explained as being more preferred or having more advantages than another embodiment or design scheme. To be precise, use of the expression "example", "for example", or the like is intended to present a relative concept in a specific manner.

In the descriptions of this application, unless otherwise specified, "a plurality of" means two or more than two. The term "and/or" in this specification describes only an association relationship between associated objects and indicates that there may be three relationships. For example, A and/or B may indicate the following three cases: Only A exists, both A and B exist, and only B exists.

Hypertension, as a serious chronic disease, increases risks of developing heart diseases, brain diseases, kidney diseases, and other diseases, and is a risk factor for cardiovascular and cerebrovascular diseases. Currently, there are various hypertension cases, for example, morning-surge hypertension that may be present in daytime and masked hypertension that may be present at night. Measurement of blood pressure in a peak time period is crucial to identification, control, and the like of hypertension.

However, human blood pressure is constantly changing. A 24-hour blood pressure curve of a human body has a specific pattern. For example, the 24-hour blood pressure curve of the human body is presented as a spoon-shaped curve of "double peaks and one valley": blood pressure is higher in 6:00 to 10:00 and 16:00 to 18:00 and reaches a valley in 0:00 to 2:00, and after that, blood pressure slightly rises and is maintained until 6:00. However, this pattern is likely to be affected by various factors such as an environment, a life status, a physical status, emotional pressure, a disease, and medicine, leading to a change in peak and valley values. In addition, due to individual differences, peak time periods of blood pressure are different.

In some related solutions, portable devices that can accurately measure blood pressure, for example, a blood pressure watch and an upper arm blood pressure monitor, mostly measure blood pressure according to oscillography. In oscillography, blood pressure may be determined based on a change in amplitude of a pressure oscillation wave in a pressure reduction process of an air cuff. For these blood pressure measurement devices, blood pressure measurement is actively initiated by a user. For example, when the user has a blood pressure measurement requirement, the user uses the blood pressure measurement device to measure blood pressure. Alternatively, the blood pressure measurement device reminds the user to measure blood pressure in the morning and evening, but a specific reminding time is set based on an empirical value or set by the user. In this case, because a time of blood pressure measurement may not be a peak time period, blood pressure in the peak time period cannot be captured, and it is difficult to discover morning-surge hypertension, masked hypertension, and the like.

In some other related solutions, a blood pressure measurement device may perform 24-hour blood pressure measurement at a fixed time interval. Generally, blood pressure is automatically measured once every 15 minutes to 30 minutes in daytime, and blood pressure is automatically measured once every 30 minutes to 60 minutes at night. In this solution, blood pressure in any time period may be obtained, and blood pressure in a peak time period may be obtained. However, oscillography is used in this solution, a watch brings a strong sense of pressure during blood pressure measurement, and frequent use causes discomfort to a user. In addition, use at night is very likely to affect sleep of the user, and consequently, measured blood pressure may not reflect a real situation of the user.

Based on this, embodiments of this application provide a blood pressure measurement method, to accurately capture blood pressure in a peak time period, resolve a problem that it is difficult to discover morning-surge hypertension, masked hypertension, and the like, and resolve a problem of discomfort caused by frequent blood pressure measurement to provide more comfortable blood pressure measurement experience to a user.

The technical solutions provided in embodiments of this application may be applied to a wearable device 100, or may be applied to a system including the wearable device 100.

Optionally, the wearable device 100 may be, for example, any device that has a blood pressure measurement function, such as a smartwatch, a smart band, a smart ankle band, a smart ring, a wireless headset, smart glasses, or a smart helmet. An operating system installed on the wearable device 100 includes but is not limited to iOS^{®}, Android^{®}, Harmony^{®}, Windows^{®}, Linux^{®}, or another operating system. In some embodiments, the wearable device 100 may be a fixed device, or may be a portable device. A specific type of the wearable device 100 and the installed operating system are not limited in this application.

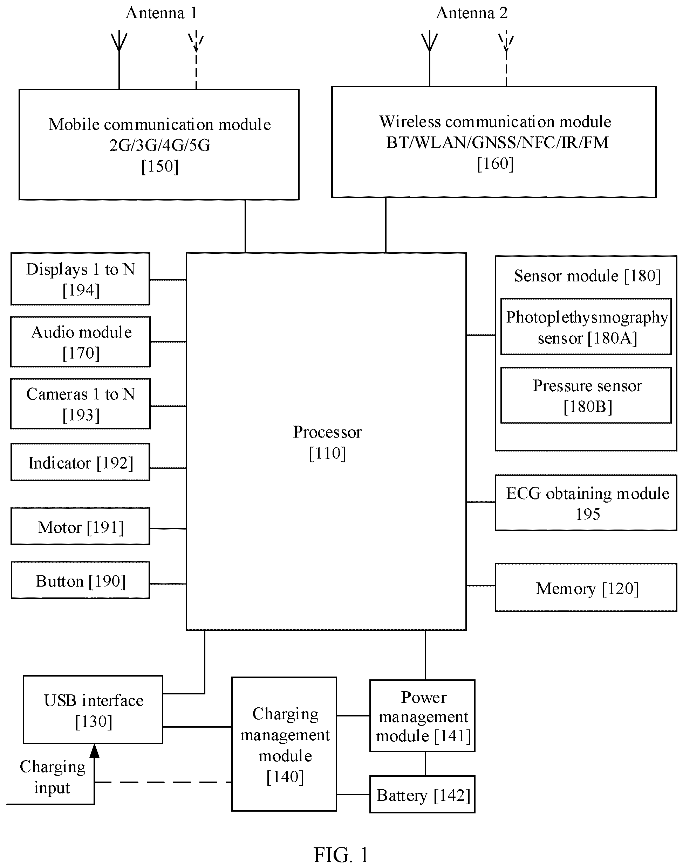

FIG. 1 is a diagram of a structure of a wearable device 100.

The wearable device 100 may include a processor 110, a memory 120, a universal serial bus (universal serial bus, USB) interface 130, a charging management module 140, a power management module 141, a battery 142, an antenna 1, an antenna 2, a mobile communication module 150, a wireless communication module 160, an audio module 170, a sensor module 180, a button 190, a motor 191, an indicator 192, a camera 193, a display 194, an electrocardiogram (Electrocardiogram, ECG) obtaining module 195, and the like. The sensor module 180 may include a photoplethysmography sensor 180A, a pressure sensor 180B, and the like.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, a neural-network processing unit (neural-network processing unit, NPU), and/or the like. Different processing units may be independent components, or may be integrated into one or more processors.

The controller may generate an operation control signal based on instruction operation code and a time sequence signal, to complete control of instruction reading and instruction execution.

A memory may be further disposed in the processor 110, and is configured to store instructions and data. In some embodiments, the memory in the processor 110 is a cache. The memory may store instructions or data just used or cyclically used by the processor 110. If the processor 110 needs to use the instructions or the data again, the processor may directly invoke the instructions or the data from the memory. This avoids repeated access, reduces waiting time of the processor 110, and improves system efficiency.

In some embodiments, the processor 110 may include one or more interfaces, for example, the USB interface 130.

The charging management module 140 is configured to receive a charging input from a charger. The charger may be a wireless charger or a wired charger.

The power management module 141 is configured to connect to the battery 142, the charging management module 140, and the processor 110. The power management module 141 receives an input from the battery 142 and/or an input from the charging management module 140, to supply power to the processor 110, the memory 120, the display 194, the camera 193, the wireless communication module 160, and the like.

A wireless communication function of the wearable device 100 may be implemented through the antenna 1, the antenna 2, the mobile communication module 150, the wireless communication module 160, and the like.

The antenna 1 and the antenna 2 are configured to transmit and receive an electromagnetic wave signal. Each antenna in the wearable device 100 may be configured to cover one or more communication frequency bands. Different antennas may be further reused, to improve antenna utilization.

The mobile communication module 150 may provide a wireless communication solution that is applied to the wearable device 100 and that includes 2G/3G/4G/5G or the like. The mobile communication module 150 may include at least one filter, a switch, a power amplifier, a low noise amplifier (low noise amplifier, LNA), and the like.

The wireless communication module 160 may provide a wireless communication solution that is applied to the wearable device 100 and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like.

In some embodiments, in the wearable device 100, the antenna 1 is coupled to the mobile communication module 150, and the antenna 2 is coupled to the wireless communication module 160, so that the wearable device 100 can communicate with a network and another device by using a wireless communication technology.

The wearable device 100 may implement a display function through the GPU, the display 194, the application processor, and the like. The GPU is a microprocessor for image processing, and is connected to the display 194 and the application processor. The GPU is configured to: perform mathematical and geometric computation, and render an image. The processor 110 may include one or more GPUs that execute program instructions to generate or change display information.

The display 194 is configured to display an image, a video, and the like. The display 194 includes a display panel. In some embodiments, the wearable device 100 may include one or N displays 194, where N is a positive integer greater than 1. In some embodiments of this application, the display 194 may be configured to output various reminder messages.

The camera 193 is configured to capture a static image or a video. In some embodiments, the wearable device 100 may include one or N cameras 193, where N is a positive integer greater than 1.

The memory 120 may be configured to store computer-executable program code. The executable program code includes instructions. The memory 120 may include a program storage area and a data storage area. The program storage area may store an operating system, an application required by at least one function (for example, a voice playing function or an image playing function), and the like. The data storage area may store data (for example, audio data or a phone book) created during use of the wearable device 100, and the like. In addition, the memory 120 may include a high-speed random access memory, and may further include a non-volatile memory, for example, at least one magnetic disk storage device, a flash storage device, or a universal flash storage (universal flash storage, UFS). The processor 110 runs instructions stored in the memory 120 and/or instructions stored in the memory disposed in the processor, to perform various function applications and data processing of the wearable device 100.

The wearable device 100 may implement an audio function, for example, music playing or recording, through the audio module 170, the application processor, and the like.

The audio module 170 is configured to convert digital audio information into an analog audio signal for output, and is also configured to convert an analog audio input into a digital audio signal. The audio module 170 may be further configured to code and decode an audio signal. The audio module 170 may include a speaker, a receiver, a microphone, an application processor, and the like, to implement an audio function. In some embodiments of this application, the audio module 170 may be further configured to output various reminder messages.

The photoplethysmography sensor 180A may obtain a PPG signal based on an LED light source and a detector by using photoplethysmography (photoplethysmography, PPG) and measuring attenuated light obtained through reflection and absorption by human blood vessels and tissues. In some embodiments of this application, the wearable device 100 may obtain user blood pressure by analyzing the PPG signal obtained by the photoplethysmography sensor 180A.

The pressure sensor 180B is configured to sense a pressure signal, and may convert the pressure signal into an electrical signal. In some embodiments, the pressure sensor 180B may be disposed on the display 194. There are a plurality of types of pressure sensors 180B, such as a resistive pressure sensor, an inductive pressure sensor, and a capacitive pressure sensor. The capacitive pressure sensor may include at least two parallel plates made of conductive materials. When force is applied to the pressure sensor 180B, capacitance between electrodes changes. The electronic device 100 determines pressure intensity based on the change in the capacitance. When a touch operation is performed on the display 194, the electronic device 100 detects intensity of the touch operation through the pressure sensor 180B. The electronic device 100 may also calculate a touch location based on a detection signal of the pressure sensor 180B. In some embodiments of this application, a signal collected by the pressure sensor 180B may be further used to determine user blood pressure.

Optionally, the sensor module 180 may further include a barometric pressure sensor, a magnetic sensor, a distance sensor, an optical proximity sensor, a gyroscope sensor, a fingerprint sensor, an ambient light sensor, a bone conduction sensor, and the like.

The button 190 includes a power button, a volume button, and the like. The button 190 may be a mechanical button, or may be a touch button.

The motor 191 may generate a vibration prompt.

The indicator 192 may be an indicator light, and may be configured to indicate a charging status and a power change, or may be configured to indicate a message, a missed call, a notification, and the like.

The ECG obtaining module 195 may be configured to obtain an electrocardiogram of a user. In some embodiments of this application, the electrocardiogram may be used to determine user blood pressure.

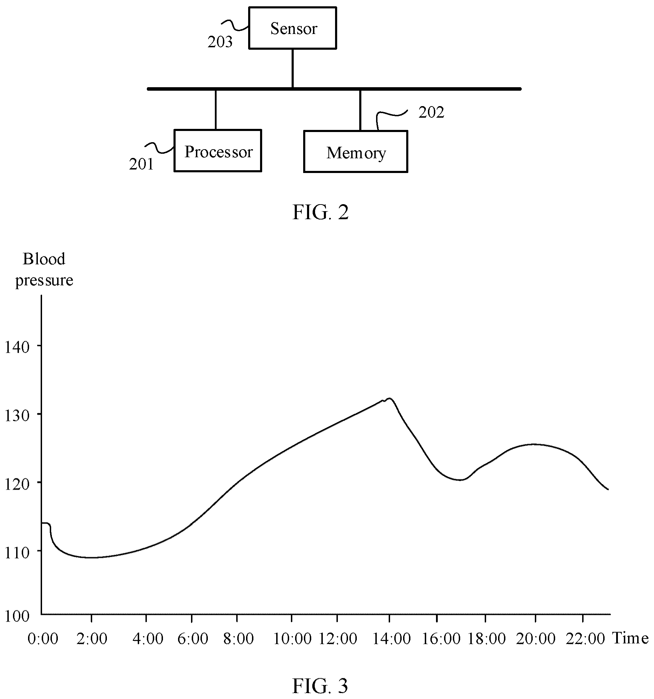

It may be understood that the foregoing is merely an example for describing the structure of the wearable device 100 in this embodiment of this application, and does not constitute a limitation on the structure and a form of the wearable device 100. The structure and the form of the wearable device 100 are not limited in embodiments of this application. For example, FIG. 2 shows another example structure of the wearable device. As shown in FIG. 2, the wearable device includes a processor 201, a memory 202, and a sensor 203. For implementation of the processor 201, the memory 202, and the sensor 203, refer to the implementation of the processor, the memory, and the sensor shown in FIG. 1.

In some other embodiments of this application, the wearable device may include more or fewer components than those shown in FIG. 1 or FIG. 2, or some components may be combined, or some components may be split, or some components may be replaced, or different component arrangements may be used. The components shown in the figure may be implemented by hardware, software, or a combination of software and hardware.

All technical solutions in the following embodiments may be implemented in a device having, for example, the structure shown in FIG. 1 or FIG. 2.

Embodiments of this application provide a blood pressure measurement method, applied to a wearable device. The wearable device may continuously monitor user blood pressure in an imperceptible manner, determine a time period of abnormal blood pressure based on the monitored user blood pressure and a monitoring time, and further measure user blood pressure in the time period of abnormal blood pressure in a perceptible manner. In this case, frequent measurement of user blood pressure in the imperceptible manner does not cause discomfort to a user, and can provide more comfortable blood pressure measurement experience to the user. Further, the time period of abnormal blood pressure may be determined based on the monitored blood pressure and the monitoring time. The time period of abnormal blood pressure may be a peak time period of blood pressure. Then, user blood pressure in the time period of abnormal blood pressure is measured in the perceptible manner. In this way, blood pressure in the peak time period can be more accurately captured, to resolve a problem that it is difficult to discover morning-surge hypertension, masked hypertension, and the like.

It may be understood that, in embodiments of this application, the imperceptible manner means that the user cannot notice or the user may slightly notice during measurement of user blood pressure. Imperceptible may also be described as slightly perceptible. Frequent blood pressure measurement in the imperceptible manner may not cause discomfort to the user, or may cause weak discomfort to the user. In some embodiments, the imperceptible manner includes but is not limited to monitoring user blood pressure by using one or more of a PPG signal, a pressure sensor signal, an electrocardiogram, and the like.

In embodiments of this application, the perceptible manner means that the user can notice during measurement of user blood pressure. Frequent measurement of user blood pressure in the perceptible manner may cause discomfort to the user, or may cause strong discomfort to the user. In some implementations, in comparison with the imperceptible manner, blood pressure can be more accurately measured in the perceptible manner. In some embodiments, the perceptible manner includes but is not limited to measuring user blood pressure by using oscillography or the like.

In some embodiments, the wearable device may continuously monitor user blood pressure in real time or periodically in daytime and/or at night in the imperceptible manner.

In the embodiments, in a possible implementation, the wearable device may automatically enable an imperceptible continuous monitoring function, to continuously monitor user blood pressure in daytime and/or at night in the imperceptible manner.

In another possible implementation, alternatively, the user may enable the imperceptible continuous monitoring function of the wearable device, and in response to the enabling operation of the user, the wearable device continuously monitors user blood pressure in daytime and/or at night in the imperceptible manner. Optionally, in this implementation, a specific time in which the wearable device continuously monitors user blood pressure in the imperceptible manner may be by default. Certainly, alternatively, the user may set an occasion for the wearable device to continuously monitor user blood pressure in the imperceptible manner. For example, the user may choose to enable only a daytime imperceptible continuous monitoring function of the wearable device, and in response to the enabling operation of the user, the wearable device continuously monitors user blood pressure in daytime in the imperceptible manner. Similarly, the user may alternatively choose to enable a night imperceptible continuous monitoring function of the wearable device, and in response to the enabling operation of the user, the wearable device continuously monitors user blood pressure at night in the imperceptible manner, and so on.

In this implementation, the wearable device may further determine whether the user is in a sleep state, and determine, according to whether the user is in the sleep state, whether a current scene is a daytime scene or a night scene. For example, if it is determined that the user is in a non-sleep state, it may be determined that the current scene is a daytime scene, and the wearable device may perform a solution of continuously monitoring user blood pressure in daytime in the imperceptible manner. If the user is in the sleep state, it may be determined that the current scene is a night scene, and the wearable device may perform a solution of continuously monitoring user blood pressure at night in the imperceptible manner. Optionally, whether the user is in the sleep state may be obtained by the wearable device by using a sleep detection function of the wearable device, or may be obtained from another device. A manner in which the wearable device obtains whether the user is in the sleep state is not limited in this application.

In still another possible implementation, alternatively, the user may directly set a time at which the imperceptible continuous monitoring function is enabled and/or a time at which the imperceptible continuous monitoring function is disabled. In response to the setting of the user, the wearable device may start to continuously monitor user blood pressure in the imperceptible manner from the enabling time set by the user, disable the imperceptible continuous monitoring function at the disabling time set by the user, and/or the like.

In some embodiments, when determining that user blood pressure monitored in the imperceptible manner satisfies a blood pressure abnormality condition, the wearable device may determine that a monitoring time period corresponding to the user blood pressure is a time period of abnormal blood pressure. It may be understood that if blood pressure satisfies the blood pressure abnormality condition, it indicates that the blood pressure is abnormal.

In some embodiments, the blood pressure abnormality condition may include at least one of a blood pressure condition, a fluctuation condition, and a quantity condition.

In a possible implementation, the blood pressure condition may include that blood pressure is not within a preset blood pressure range (for example, the preset blood pressure range may include greater than or equal to a preset blood pressure threshold), and/or that a blood pressure curve does not match a preset curve (or the condition may be described as that a blood pressure change pattern does not match a preset pattern).

It may be understood that, if blood pressure monitored in a time period is not within the preset blood pressure range, it may indicate that some or all blood pressure monitored in the time period is not within the preset blood pressure range. Alternatively, it may indicate that a statistical value such as an average value, a mode, or a median of all the blood pressure monitored in the time period is not within the preset blood pressure range. Alternatively, it may indicate that a quantity of blood pressure that is not within the preset blood pressure range and that is monitored in the time period is greater than or equal to a preset quantity threshold, or may indicate that a proportion of blood pressure that is not within the preset blood pressure range and that is monitored in the time period to all the blood pressure monitored in the time period is within a first preset proportion range (for example, the first preset proportion range may include greater than or equal to a first preset proportion threshold), or the like.

For example, the preset blood pressure range may be greater than or equal to 90 millimeters of mercury (mmHg) and less than or equal to 140 mmHg according to a medical definition, or may be set based on an empirical value, a statistical value, or the like. For example, statistics on blood pressure of different users in each time period are collected, to obtain a preset blood pressure range corresponding to each time period. Optionally, preset blood pressure ranges corresponding to different time periods may be the same or may be different.

For example, the preset curve may be a blood pressure curve obtained by collecting statistics on blood pressure in a period of time. Optionally, all time periods in which blood pressure is monitored may have corresponding preset curves, and the preset curves may be different. For example, a time period is 24 hours, and a blood pressure curve (namely, a preset curve) of the time period may be, for example, a spoon-shaped blood pressure curve. FIG. 3 is a diagram of a spoon-shaped blood pressure curve according to an embodiment of this application. The blood pressure curve has a peak and a valley like a spoon, and presents a change pattern of double peaks and one valley.

In a possible implementation, the fluctuation condition may include that a fluctuation value of blood pressure is not within a preset fluctuation range (for example, the preset fluctuation range may include less than or equal to a preset fluctuation threshold). For example, a fluctuation value of blood pressure in a time period may be determined by calculating various values such as a variance, an average value, and a difference of blood pressure in the time period. It may be understood that, when blood pressure monitored in a time period satisfies the fluctuation condition, it may indicate that the blood pressure in the time period may fluctuate abnormally.

In some implementations, the fluctuation condition may further specifically include a condition of small fluctuation and a condition of large fluctuation. For example, the condition of small fluctuation may include that a fluctuation value of blood pressure is within a first fluctuation range, and the condition of large fluctuation may include that a fluctuation value of blood pressure is within a second fluctuation range. For example, the first fluctuation range may include greater than or equal to a first fluctuation threshold and less than or equal to a second fluctuation threshold, and the second fluctuation range may include greater than the second fluctuation threshold.

It may be understood that if blood pressure monitored in a time period satisfies the condition of small fluctuation, it may indicate that fluctuation of the blood pressure in the time period may be small. If blood pressure monitored in a time period satisfies the condition of large fluctuation, it may indicate that fluctuation of the blood pressure in the time period may be large.

In a possible implementation, the quantity condition may include: In a first time period in which blood pressure is continuously monitored in the imperceptible manner, for a time period, a quantity of times blood pressure monitored in the time period satisfies the blood pressure condition and/or the fluctuation condition is within a first quantity range (for example, the first quantity range may include greater than or equal to a preset quantity-of-times threshold), or a proportion of a quantity of time periods in which monitored blood pressure satisfies the blood pressure condition and/or the fluctuation condition to a quantity of time periods included in the first time period is within a first proportion range.

For example, the first time period includes but is not limited to n daytimes, n nights, n daytimes and n nights, or the like, where n is greater than 0. Optionally, the first time period may be a consecutive period of time, for example, n consecutive daytimes, or may not be a consecutive period of time.

Optionally, in embodiments of this application, the first time period may include one or more time periods. For example, the first time period is three daytimes, and each daytime is 6:00 to 18:00. If the 12 hours are divided into 12 time periods, the time periods included in the first time period may include: 6:00 to 7:00, 7:00 to 8:00, 8:00 to 9:00, and so on.

It may be understood that, in the foregoing example, a length of the time period is 1 hour, and the time period may alternatively be of another length, for example, 2 hours or 3 hours. A length of each time period is not limited in embodiments of this application. Optionally, lengths of the time periods may be the same or may be different.

It may be further understood that, in the foregoing example, time points corresponding to the time periods are consecutive, and time points corresponding to different time periods may alternatively be non-consecutive. For example, the time periods included in the first time period may include: 6:00 to 8:00, 9:00 to 10:00, 12:00 to 13:00, and the like. Optionally, time intervals between different time periods may be the same or may be different.

It may be understood that division into time periods is not limited in embodiments of this application.

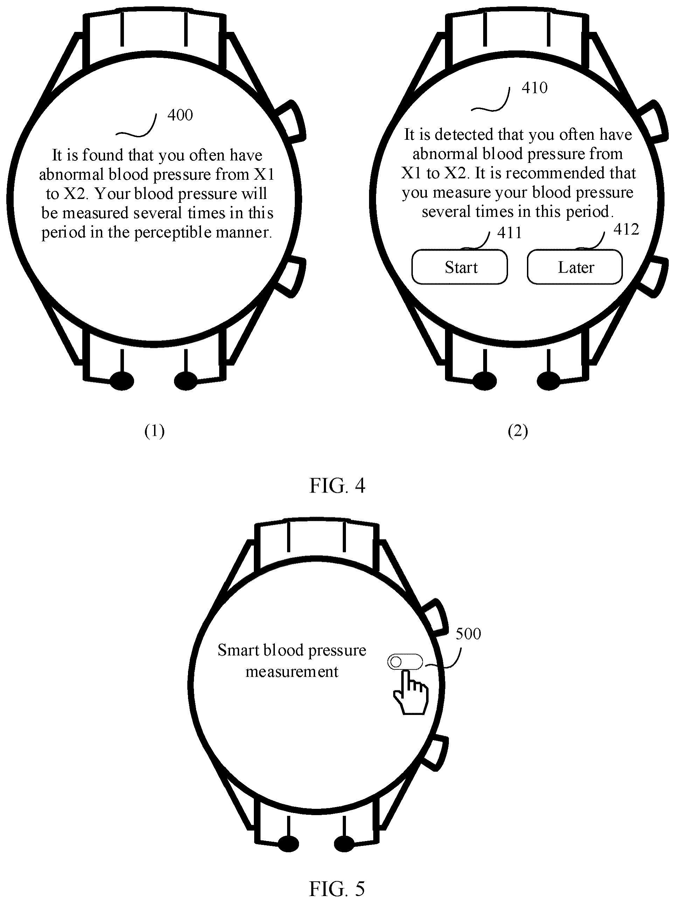

In some embodiments, after determining the time period of abnormal blood pressure, the wearable device may automatically start the perceptible manner to measure user blood pressure in the time period of abnormal blood pressure. Optionally, before or after automatically starting the perceptible manner to measure the user blood pressure in the time period of abnormal blood pressure, the wearable device may further output, for example, a reminder message 400 shown in (1) in FIG. 4, to remind the user that the perceptible manner is to be started to measure blood pressure in the time period because blood pressure is monitored to be abnormal.

It may be understood that interface diagrams of the wearable device shown in embodiments of this application are all based on an example in which the wearable device is a smartwatch.

In some other embodiments, after determining the time period of abnormal blood pressure, the wearable device may remind the user (for example, a display displays a reminder message or a reminder message is broadcast by using a voice) to start the perceptible manner to measure the user blood pressure in the time period of abnormal blood pressure. For example, the wearable device may output, for example, a reminder message 410 shown in (2) in FIG. 4, to remind the user to use, in the time period of abnormal blood pressure, the perceptible manner to measure the user blood pressure in the time period of the abnormality.

In the embodiments, in a possible implementation, the user may start the perceptible manner of the wearable device to measure the user blood pressure in the time period of abnormal blood pressure. In this implementation, a device monitoring user blood pressure in the imperceptible manner and a device measuring the user blood pressure in the time period of abnormal blood pressure in the perceptible manner are the same wearable device. Optionally, the wearable device may display, for example, a start button 411 shown in (2) in FIG. 4. The wearable device detects, for example, a tap operation of the user on the start button 411, and in response to the operation, the wearable device measures the user blood pressure in the time period of abnormal blood pressure (for example, 9:00 to 10:00) in the perceptible manner. Certainly, the user may alternatively use another button operation, a voice operation, a gesture operation, or the like to start the perceptible manner of the wearable device to measure the user blood pressure in the time period of abnormal blood pressure.

Optionally, the wearable device may further display, for example, a later button 412 shown in (2) in FIG. 4. The wearable device detects, for example, a tap operation of the user on the later button 412, and in response to the operation, the wearable device may first skip an operation of measuring the user blood pressure in the time period of abnormal blood pressure in the perceptible manner.

In another possible implementation, the user may use another blood pressure measurement device that can use a perceptible manner (for example, an upper arm blood pressure monitor) to measure the user blood pressure in the time period of abnormal blood pressure. In this implementation, the wearable device monitoring user blood pressure in the imperceptible manner and a device measuring the user blood pressure in the time period of abnormal blood pressure in the perceptible manner are different devices. Optionally, in this implementation, the wearable device may also present, for example, an interface shown in (2) in FIG. 4.

In still some other embodiments, the wearable device may further send a notification message to another blood pressure measurement device that can use a perceptible manner and that is worn by the user. Correspondingly, after receiving the notification message, the another blood pressure measurement device may start the perceptible manner to measure the user blood pressure in the time period of abnormal blood pressure.

Optionally, in the foregoing embodiment, the wearable device may further output a reminder message in the time period of abnormal blood pressure, to remind the user to perform blood pressure measurement in the perceptible manner in the time period.

Optionally, in embodiments of this application, a reminder message that is output by the wearable device and that is used to remind the user to measure the user blood pressure in the time period of abnormal blood pressure in the perceptible manner, for example, the reminder message 400 shown in (1) in FIG. 4 or the reminder message 410 shown in (2) in FIG. 4, may include a specific reason (not shown in the figure) for abnormal blood pressure, for example, abnormal high pressure or abnormal fluctuation, so that the user learns of a specific situation of the abnormal blood pressure.

It may be understood that, in embodiments of this application, messages output by the wearable device include but are not limited to, for example, the reminder message 400 shown in (1) in FIG. 4 and the reminder message 410 shown in (2) in FIG. 4, or may be sent by the wearable device to another device (for example, a mobile phone), and the another device outputs a corresponding message.

In some embodiments, when measuring the user blood pressure in the time period of abnormal blood pressure in the perceptible manner, the wearable device may measure the user blood pressure at different frequency in the time period of abnormal blood pressure.

In a possible implementation, when determining that the user blood pressure monitored in the imperceptible manner satisfies the condition of small fluctuation, the wearable device determines that the monitoring time corresponding to the user blood pressure is the time period of abnormal blood pressure. Further, when the user blood pressure in the time period of abnormal blood pressure is measured in the perceptible manner, the user blood pressure may be measured at low frequency (for example, once every 10 minutes). In this case, because fluctuation of blood pressure in the time period of abnormal blood pressure is small, real blood pressure of the user can be reflected when blood pressure measurement is performed a small quantity of times, so that power consumption of the wearable device can be reduced when blood pressure in a peak time period is accurately captured.

In another possible implementation, when determining that the user blood pressure monitored in the imperceptible manner satisfies the condition of large fluctuation, the wearable device determines that the monitoring time corresponding to the user blood pressure is the time period of abnormal blood pressure. Further, when the user blood pressure in the time period of abnormal blood pressure is measured in the perceptible manner, the user blood pressure may be measured at high frequency (for example, once every 5 minutes). In this case, because fluctuation of blood pressure in the time period of abnormal blood pressure is large, real blood pressure of the user can be reflected only when blood pressure measurement is performed a large quantity of times. In this way, blood pressure in a peak time period can be accurately captured.

Optionally, in embodiments of this application, when blood pressure measurement frequency is greater than or equal to a preset frequency threshold, the blood pressure measurement frequency is high frequency; or when blood pressure measurement frequency is less than the preset frequency threshold, the frequency is low frequency. The preset frequency threshold may be, for example, once every 8 minutes or other frequency, and may be set by a developer according to an actual requirement.

In some embodiments, when determining that the user blood pressure monitored in the imperceptible manner satisfies the blood pressure abnormality condition, the wearable device may further receive, before measuring the user blood pressure in the time period of abnormal blood pressure in the perceptible manner, an operation of enabling a smart blood pressure measurement function by the user. For example, as shown in FIG. 5, the wearable device displays an enable button 500 of the smart blood pressure measurement function, and the user may enable the smart blood pressure measurement function by using the enable button 500. For example, after the wearable device receives, for example, a tap operation of the user on the enable button 500, in response to the operation, the wearable device measures the user blood pressure in the time period of abnormal blood pressure in the perceptible manner when determining that the user blood pressure monitored in the imperceptible manner satisfies the blood pressure abnormality condition.

With reference to several scenarios, the following specifically describes the technical solutions provided in the embodiments of this application by using an example in which the blood pressure abnormality condition includes at least the blood pressure condition and/or the fluctuation condition.

Scenario 1: The wearable device continuously monitors user blood pressure in the first time period (for example, n daytimes) in daytime in the imperceptible manner.

In some embodiments, if user blood pressure monitored in any daytime time period does not satisfy the blood pressure abnormality condition, it may be determined that user blood pressure is normal. In other words, there is no time period of abnormal blood pressure in daytime. In the embodiments, the wearable device may not start the perceptible manner to measure user blood pressure.

For example, in embodiments of this application, that user blood pressure does not satisfy the blood pressure condition may mean that the user blood pressure does not satisfy at least one of the blood pressure condition, the fluctuation condition, and the quantity condition.

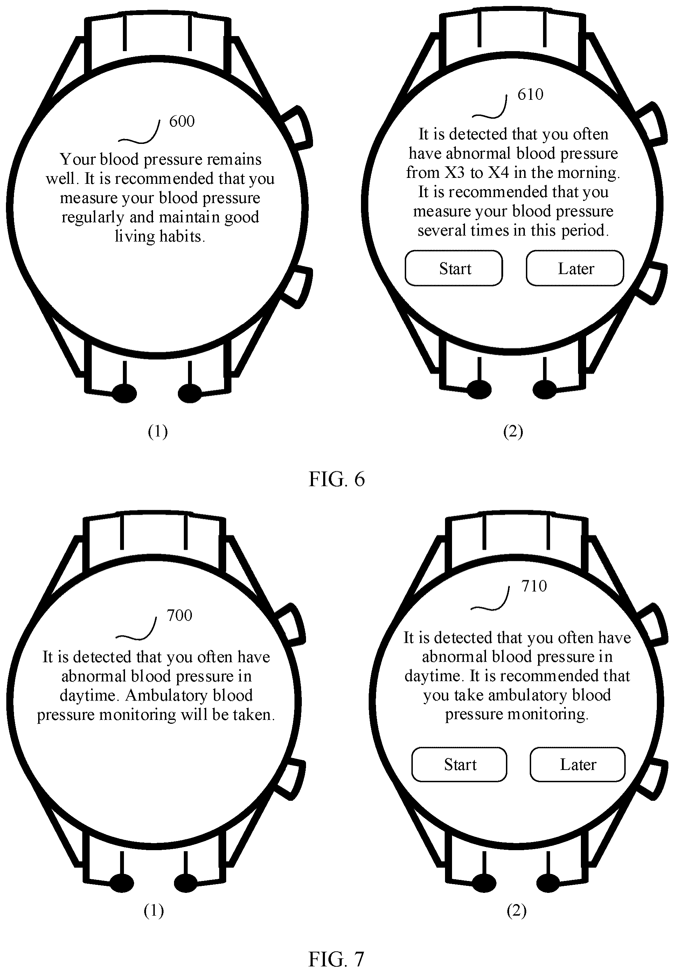

Optionally, in the embodiments, the wearable device may output, for example, a reminder message 600 shown in (1) in FIG. 6, to remind the user that blood pressure is normal.

In some other embodiments, if user blood pressure monitored in a daytime time period satisfies the blood pressure condition and/or the fluctuation condition, or satisfies the blood pressure condition and/or the fluctuation condition and further satisfies the quantity condition, it may be determined that the daytime time period is a time period of abnormal blood pressure. In the embodiments, the wearable device may start the perceptible manner to measure user blood pressure in the time period of abnormal blood pressure.

Optionally, in the embodiments, the wearable device may further output, for example, the reminder message 400 shown in (1) in FIG. 4 or the reminder message 410 shown in (2) in FIG. 4.

For example, the first time period is four daytimes, and a length of each time period included in each daytime is 1 hour. Table 1 shows some examples of time periods in which the blood pressure condition and/or the fluctuation condition are/is satisfied according to an embodiment of this application.

**Table 1**

| Monitoring time | Time period in which the blood pressure condition and/or the fluctuation condition are/is satisfied |
|---|---|
| First daytime | 9:00 to 10:00 |
| Second daytime | 9:00 to 10:00, 14:00 to 15:00 |
| Third daytime | 9:00 to 10:00 |
| Fourth daytime | 9:00 to 10:00 |

For example, as shown in Table 1, in 9:00 to 10:00 and 14:00 to 15:00, the blood pressure condition and/or the fluctuation condition are/is satisfied. In this case, it is determined that both 9:00 to 10:00 and 14:00 to 15:00 are time periods of abnormal blood pressure.

For another example, the quantity condition is as follows: In the first time period, a quantity of times blood pressure monitored in a time period satisfies the blood pressure condition and/or the fluctuation condition is within the first quantity range, where the first quantity range is three times. As shown in Table 1, in 9:00 to 10:00 and 14:00 to 15:00, the blood pressure condition and/or the fluctuation condition are/is satisfied, and in 9:00 to 10:00, a quantity of times the blood pressure condition and/or the fluctuation condition are/is satisfied is 4 and is within the first quantity range. In this case, it is determined that 9:00 to 10:00 is a time period of abnormal blood pressure. In 14:00 to 15:00, a quantity of times the blood pressure condition and/or the fluctuation condition are/is satisfied is 1 and is not within the first quantity range. In this case, it is determined that 14:00 to 15:00 is not a time period of abnormal blood pressure.

In some scenarios, in consideration of possible presence of morning-surge hypertension in daytime, in a specific embodiment, if user blood pressure monitored in a time period in the morning in daytime satisfies the blood pressure condition and/or the fluctuation condition, or satisfies the blood pressure condition and/or the fluctuation condition and further satisfies the quantity condition, it may be determined that the time period in the morning is a time period of abnormal blood pressure, and morning-surge hypertension may be present in the time period of abnormal blood pressure. In this embodiment, the wearable device may also start the perceptible manner to measure user blood pressure in the time period of abnormal blood pressure. In this way, the time period of abnormal blood pressure in the morning is captured, where morning-surge hypertension may be present in the time period of abnormal blood pressure, and further, user blood pressure is measured in the perceptible manner in the time period of abnormal blood pressure in the morning, to resolve a problem that it is difficult to discover morning-surge hypertension.

Optionally, in this embodiment, the wearable device may output, for example, a reminder message 610 shown in (2) in FIG. 6. For descriptions of another button shown in (2) in FIG. 6, refer to descriptions of a corresponding button shown in (2) in FIG. 4.

Optionally, in the foregoing embodiments of Scenario 1, when measuring the user blood pressure in the time period of abnormal blood pressure in the perceptible manner, the wearable device may measure the user blood pressure at high frequency. In this case, because the user blood pressure is measured in a daytime scene and the user is in an awake state, when the user blood pressure is measured in the perceptible manner, the measurement at the high frequency causes weak discomfort to the user. In this way, a physical status of the user is not affected, and a more accurate blood pressure situation of the user can be obtained.

In still some other embodiments, a quantity of time periods of abnormal blood pressure included in a daytime is greater than or equal to a first threshold, or a total length of a time period of abnormal blood pressure included in a daytime is within a first length range (for example, the first length range may be greater than or equal to a first length threshold), or a proportion of a total length of a time period of abnormal blood pressure in a daytime to a total length of a time in which blood pressure is monitored in the imperceptible manner in the daytime is within a second proportion range (for example, the second proportion range may be greater than or equal to a first proportion), or a proportion of a quantity of time periods of abnormal blood pressure in a daytime to a total quantity of time periods in which blood pressure is monitored in the imperceptible manner in the daytime is within a third proportion range (for example, the third proportion range may be greater than or equal to a second proportion). Optionally, a quantity of daytimes satisfying the foregoing condition is further within a second quantity range (for example, the second quantity range may be greater than or equal to a second threshold), or a proportion of the quantity of daytimes satisfying the foregoing condition to the first time period is further greater than or equal to a third proportion.

Alternatively, a quantity of time periods of abnormal blood pressure in the first time period is greater than or equal to a third threshold, or a total length of a time period of abnormal blood pressure in the first time period is greater than or equal to a second length threshold, or a proportion of the total length of the time period of abnormal blood pressure in the first time period to the first time period is greater than or equal to a fourth proportion, or a proportion of the quantity of time periods of abnormal blood pressure in the first time period to total time periods included in the first time period is greater than or equal to a fifth proportion.

In this case, it indicates that the user often has blood pressure abnormalities throughout the daytime. In the embodiments, when measuring user blood pressure in the perceptible manner, the wearable device may start an ambulatory blood pressure monitoring mode.

It may be understood that the ambulatory blood pressure monitoring mode may mean that the wearable device may continuously monitor user blood pressure in each time period in real time or periodically in the perceptible manner. In other words, in addition to monitoring the user blood pressure in the time period of abnormal blood pressure in the perceptible manner, the wearable device further monitors user blood pressure in a time period of normal blood pressure. Optionally, measurement frequency of the ambulatory blood pressure monitoring mode may be greater than, less than, or equal to the foregoing high frequency, or greater than the foregoing low frequency.

Optionally, in the embodiments, the wearable device may further output, for example, a reminder message 700 shown in (1) in FIG. 7 or a reminder message 710 shown in (2) in FIG. 7. For descriptions of another button shown in (2) in FIG. 7, refer to descriptions of a corresponding button shown in (2) in FIG. 4.

For example, the first time period is still four daytimes. Table 2 shows some examples of total lengths of some time periods of abnormal blood pressure according to an embodiment of this application.

**Table 2**

| Monitoring time | Total length of a time period of abnormal blood pressure (proportion) |
|---|---|
| First daytime | 2 hours (20%) |
| Second daytime | 3.5 hours (40%) |
| Third daytime | 3 hours (35%) |
| Fourth daytime | 2 hours (30%) |

It may be understood that the proportion shown in Table 2 is a proportion of a total length of a time period of abnormal blood pressure included in a daytime to a total length of a time in which blood pressure is monitored in the imperceptible manner in the daytime.

For example, the first length range is greater than or equal to 1.5 hours, and the second quantity range is greater than or equal to 2. A total length of a time period of abnormal blood pressure included in each of the first daytime to the fourth daytime is within the first length range, and a quantity of daytimes in which the first length range is satisfied is 4 and is within the second quantity range. In this case, it indicates that the user often has blood pressure abnormalities throughout the daytime. Therefore, when measuring the user blood pressure in the time period of abnormal blood pressure in the perceptible manner, the wearable device may start the ambulatory blood pressure monitoring mode.

For another example, the second proportion range is greater than or equal to 25%, and the second quantity range is greater than or equal to 2. A proportion of a total length of a time period of abnormal blood pressure included in each of the second daytime to the fourth daytime to a total length of a time in which blood pressure is monitored in the imperceptible manner in the daytime is within the second proportion range, and a quantity of daytimes in which the second proportion range is satisfied is 3 and is within the second quantity range. In this case, it indicates that the user often has blood pressure abnormalities throughout the daytime. Therefore, when measuring the user blood pressure in the time period of abnormal blood pressure in the perceptible manner, the wearable device may start the ambulatory blood pressure monitoring mode.

According to the solutions in the scenario, user blood pressure in daytime is monitored in the imperceptible or slightly perceptible manner, to capture a peak time period of blood pressure, and then user blood pressure in the peak time period is accurately measured in the perceptible manner based on the captured peak time period. In this way, the blood pressure in the peak time period can be more accurately captured when a quantity of times of measuring blood pressure in the perceptible manner is reduced, to resolve a problem that it is difficult to discover morning-surge hypertension, masked hypertension, and the like. In addition, because the quantity of times of measuring user blood pressure in the perceptible manner is reduced, device power consumption can be reduced, and more comfortable blood pressure measurement experience can be provided to the user.

Scenario 2: The wearable device continuously monitors user blood pressure in the first time period (for example, n nights) at night in the imperceptible manner.

In some embodiments, if user blood pressure monitored in any night time period does not satisfy the blood pressure abnormality condition, it may be determined that user blood pressure is normal. In other words, there is no time period of abnormal blood pressure at night. In the embodiments, the wearable device may not start the perceptible manner to measure user blood pressure.

Optionally, in the embodiments, the wearable device may also output, for example, the reminder message 600 shown in (1) in FIG. 6, to remind the user that blood pressure is normal.

In some other embodiments, if user blood pressure monitored in a night time period satisfies the blood pressure condition and/or the fluctuation condition, or the user blood pressure monitored in the time period satisfies the blood pressure condition and/or the fluctuation condition and further satisfies the quantity condition, it may be determined that the time period is a time period of abnormal blood pressure. In the embodiments, the wearable device may start the perceptible manner to measure user blood pressure in the time period of abnormal blood pressure.

Optionally, in the embodiments, the wearable device may also output, for example, the reminder message 400 shown in (1) in FIG. 4 or the reminder message 410 shown in (2) in FIG. 4. Optionally, to avoid affecting the user in a night scene, the wearable device may output the reminder message in daytime, or may not output the reminder message and directly and automatically start the perceptible manner to measure the user blood pressure in the time period of abnormal blood pressure.

In the embodiments, in a possible implementation, if user blood pressure monitored in a night time period satisfies the condition of small fluctuation of blood pressure, the wearable device may determine that the time period is a time period of abnormal blood pressure. Optionally, in this implementation, user blood pressure monitored in the time period of abnormal blood pressure may further satisfy one or more of the blood pressure condition and the quantity condition. In this implementation, when measuring the user blood pressure in the time period of abnormal blood pressure in the perceptible manner, the wearable device may measure the user blood pressure at low frequency. In this case, because fluctuation of blood pressure in the time period of abnormal blood pressure is small, real blood pressure of the user can be reflected when blood pressure measurement is performed a small quantity of times, so that power consumption of the wearable device can be reduced when blood pressure in a peak time period is accurately captured.

In another possible implementation, if user blood pressure monitored in a night time period satisfies the condition of large fluctuation, the wearable device may determine that the time period is a time period of abnormal blood pressure. Optionally, in this implementation, user blood pressure monitored in the time period of abnormal blood pressure may further satisfy one or more of the blood pressure condition and the quantity condition. In this implementation, when measuring the user blood pressure in the time period of abnormal blood pressure in the perceptible manner, the wearable device may measure the user blood pressure at high frequency. In this case, because fluctuation of blood pressure in the time period of abnormal blood pressure is large, real blood pressure of the user can be reflected only when blood pressure measurement is performed a large quantity of times. In this way, blood pressure in a peak time period can be accurately captured.

Optionally, in the embodiments, the wearable device may also output, for example, the reminder message shown in FIG. 4. Optionally, the wearable device may further output frequency at which blood pressure is measured in the perceptible manner, and the like.

In still some other embodiments, in consideration of a high risk of presence of masked hypertension at night, if user blood pressure monitored in a night time period satisfies the blood pressure condition and/or the fluctuation condition, or the user blood pressure monitored in the time period satisfies the blood pressure condition and/or the fluctuation condition and further satisfies the quantity condition, it may be determined that the time period is a time period of abnormal blood pressure. Further, when determining that there is a time period of abnormal blood pressure at night, the wearable device may start the perceptible manner to measure user blood pressure throughout a night. Alternatively, an entire night is directly determined as a time period of abnormal blood pressure. This can increase a probability of capturing masked hypertension.

In still some other embodiments, if there is a time period of abnormal blood pressure at night, and a proportion of a quantity of such nights to a quantity of nights included in the first time period is greater than or equal to a sixth proportion, or the quantity of such nights is greater than or equal to a fourth threshold, the wearable device may measure user blood pressure throughout a night in the perceptible manner.

In still some other embodiments, the wearable device may also use the solution in the embodiment shown in FIG. 7 in Scenario 1. For example, when a quantity of time periods of abnormal blood pressure and/or a total length of a time period of abnormal blood pressure satisfy/satisfies a specific condition, the perceptible manner is started to measure user blood pressure throughout a night.

Optionally, in the foregoing embodiment in which the user blood pressure is measured throughout the night in the perceptible manner, when the wearable device starts the perceptible manner to measure the user blood pressure throughout the night, similar to the second embodiment described in this scenario, the wearable device may also measure the user blood pressure throughout the night at low frequency or high frequency. Certainly, the wearable device may alternatively measure user blood pressure throughout a night in the ambulatory blood pressure monitoring mode.

Optionally, user blood pressure satisfying the condition of large fluctuation or the condition of small fluctuation may be user blood pressure monitored in several time periods included in a night, or may be user blood pressure monitored throughout a night.

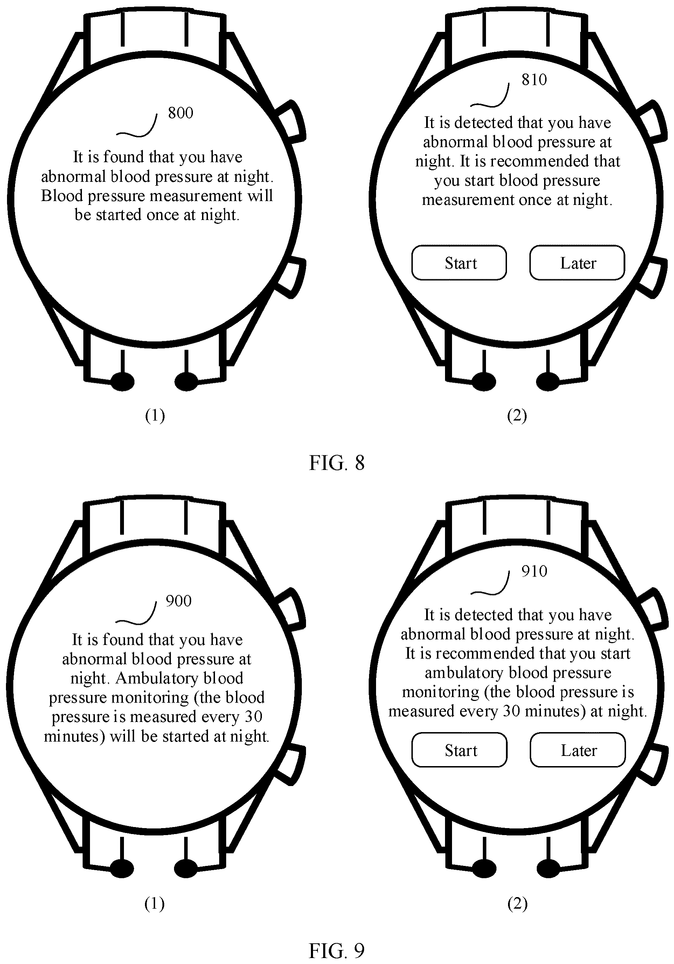

For example, blood pressure is measured once at low frequency throughout a night. When the wearable device measures user blood pressure at night in the perceptible manner at the low frequency, the wearable device may output, for example, a reminder message 800 shown in (1) in FIG. 8 or a reminder message 810 shown in (2) in FIG. 8.

For example, measurement frequency of the ambulatory blood pressure monitoring mode is once every 30 minutes. When the wearable device measures user blood pressure at night in the perceptible manner in the ambulatory blood pressure monitoring mode, the wearable device may output, for example, a reminder message 900 shown in (1) in FIG. 9 or a reminder message 910 shown in (2) in FIG. 9.

As shown in FIG. 8 and FIG. 9, when reminding the user to start the perceptible manner to measure user blood pressure at night, the wearable device may further remind the user of measurement frequency and the like.

It may be understood that, in the foregoing embodiments, the solutions for measuring user blood pressure throughout a night are all determined by determining a time period of abnormal blood pressure at night. Alternatively, whether to measure user blood pressure throughout a night in the perceptible manner may be directly determined according to whether user blood pressure monitored throughout a night in the imperceptible manner satisfies the blood pressure abnormality condition. In other words, an entire night is used as a time period. For specific implementations, refer to related implementations of the foregoing time period. For example, if the user blood pressure monitored throughout the night in the imperceptible manner satisfies the blood pressure abnormality condition, the wearable device may measure user blood pressure throughout a night in the perceptible manner. Otherwise, the wearable device may not measure user blood pressure throughout a night in the perceptible manner.

For example, the first time period is four nights. Table 3 shows examples of whether the blood pressure condition is satisfied and fluctuation of blood pressure according to an embodiment of this application.

**Table 3**

| Monitoring time | Whether the blood pressure condition is satisfied | Fluctuation |
|---|---|---|
| First night | Yes | The condition of large fluctuation is satisfied |
| Second night | Yes | The condition of large fluctuation is satisfied |
| Third night | Yes | The condition of large fluctuation is satisfied |
| Fourth night | No | The fluctuation condition is not satisfied |

As shown in Table 3, for example, user blood pressure monitored at the first night, the second night, and the third night satisfies the condition of large fluctuation and further satisfies the blood pressure condition. In this case, the wearable device may measure user blood pressure throughout a night in the perceptible manner at high frequency. For another example, the sixth proportion is 50%. A proportion of a quantity of nights at which the blood pressure condition and/or the condition of large fluctuation are/is satisfied to a quantity of nights included in the first time period is 75%, which is greater than the sixth proportion. In this case, the wearable device may measure user blood pressure throughout a night in the perceptible manner at high frequency.

According to the solutions in the scenario, user blood pressure at night is monitored in the imperceptible or slightly perceptible manner, to preliminarily obtain, through screening, a user having abnormal blood pressure at night, and then user blood pressure is accurately measured in the perceptible manner. In this way, blood pressure in a peak time period can be more accurately captured when a quantity of times of measuring blood pressure in the perceptible manner is reduced, to resolve a problem that it is difficult to discover masked hypertension and the like, avoid excessive disturbance to sleep of the user, and provide more comfortable blood pressure measurement experience to the user.

In some embodiments, after monitoring user blood pressure in the imperceptible manner, the wearable device may output details of the monitored blood pressure. For example, the details of the blood pressure may include but are not limited to blood pressure monitored each time, blood pressure monitored throughout a daytime and/or a night, blood pressure monitored in a preset time period, various statistics of blood pressure, and the like. Certainly, the wearable device may send the monitored blood pressure to another device, and the another device outputs details of blood pressure and the like.

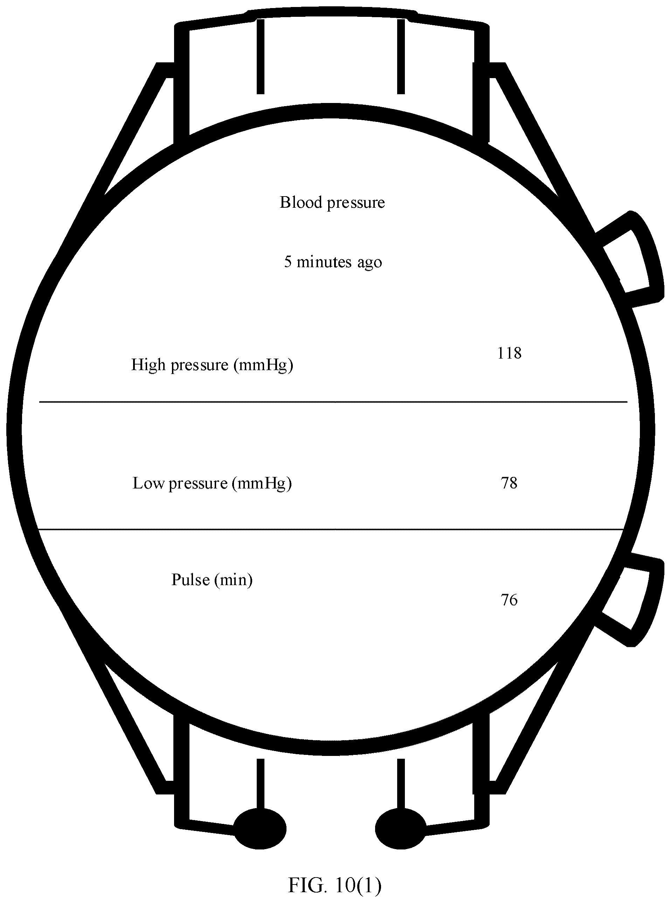

For example, the wearable device outputs blood pressure monitored each time. FIG. 10(1) shows an example of details of blood pressure according to an embodiment of this application. As shown in FIG. 10(1), the details of the blood pressure may include one or more of monitored high pressure, monitored low pressure, a monitored pulse, a monitoring time (for example, 5 minutes ago), and the like.

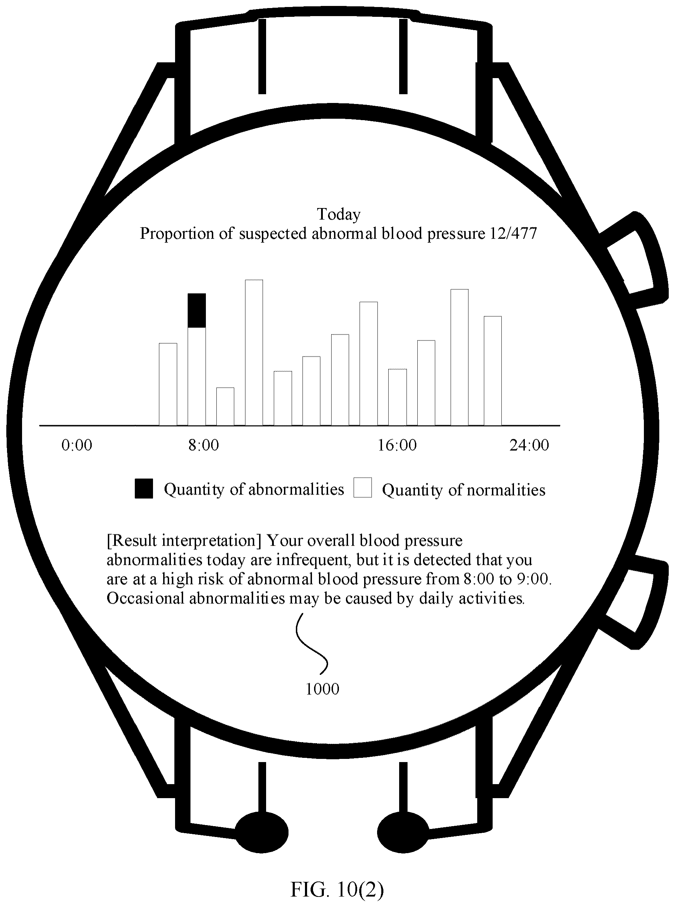

For example, the wearable device outputs statistics of blood pressure monitored throughout a daytime. FIG. 10(2) shows another example of details of blood pressure according to an embodiment of this application.

For example, a length of each time period included in a daytime is 1 hour. As shown in FIG. 10(2), the wearable device may output one or more of a measured blood pressure value, a quantity of measured blood pressure values (this is used as an example in FIG. 9), and the like in each time period, for example, 8:00 to 9:00 or 9:00 to 10:00. Optionally, the wearable device may further output a proportion of abnormal blood pressure, for example, a proportion of 12/477 shown in FIG. 10(2). Certainly, the wearable device may further mark one or more of abnormal blood pressure, normal blood pressure, a specific type of abnormal blood pressure (for example, abnormal high pressure or abnormal fluctuation), and the like.

Optionally, the wearable device may further output a result interpretation 1000, so that the user learns of a reason for abnormal blood pressure and the like.

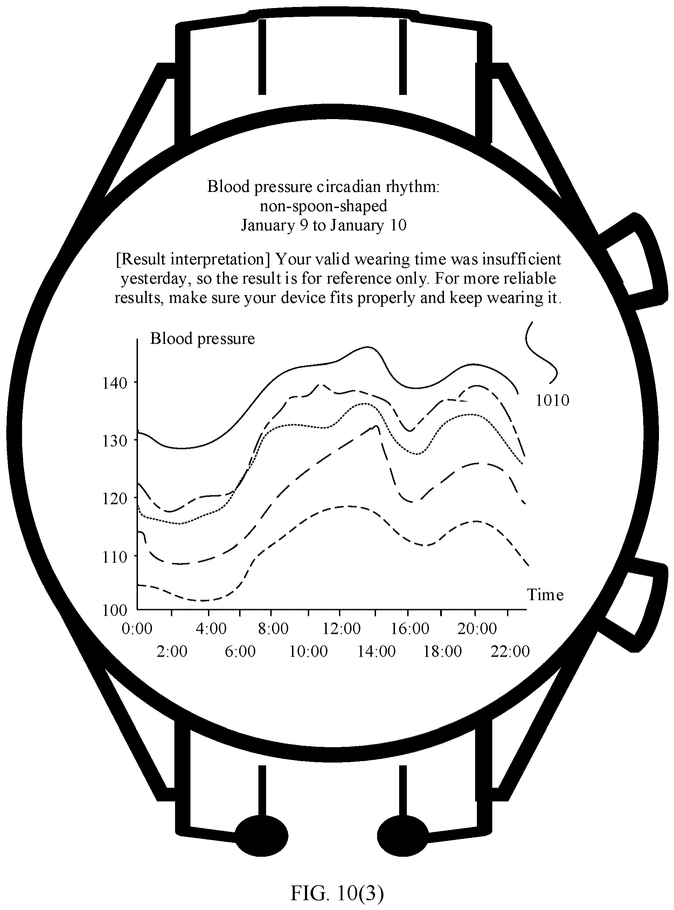

For example, the wearable device outputs statistics of blood pressure in a preset time period. FIG. 10(3) shows still another example of details of blood pressure according to an embodiment of this application.

FIG. 10(3) shows five blood pressure curves, and each blood pressure curve includes blood pressure values measured throughout a daytime and a night. Optionally, the wearable device may further output fluctuation of measured blood pressure, for example, "Blood pressure circadian rhythm: non-spoon-shaped", a blood pressure measurement time, for example, "January 9 to January 10", and the like. Optionally, the wearable device may further output a result interpretation 1010, so that the user learns of a situation of blood pressure.

It may be understood that, in embodiments of this application, details of blood pressure may be presented in various forms such as a curve graph, a pie chart, a bar chart, and a table. This is not limited in this application.

It may be understood that, in the foregoing embodiments, the following is used as an example: blood pressure is measured in the perceptible manner when blood pressure monitored in the imperceptible manner is abnormal. Alternatively, blood pressure may be measured in the perceptible manner on another occasion. For example, the wearable device may set, by default, a time, frequency, and the like for measuring blood pressure in the perceptible manner, or the user may set a time, frequency, and the like for measuring blood pressure in the perceptible manner.

The foregoing mainly describes the solutions provided in embodiments of this application from the perspective of the method. It may be understood that, to implement the foregoing functions, the wearable device includes a corresponding hardware structure and/or a corresponding software module for performing each function. Units and algorithm steps in the examples described with reference to embodiments disclosed in this application can be implemented in a form of hardware or a combination of hardware and computer software in embodiments of this application. Whether a function is performed by hardware or hardware driven by a computer depends on particular applications and design constraints of the technical solutions. A person skilled in the art may use different methods to implement the described functions for each particular application, but it shall not be considered that the implementation goes beyond the scope of the technical solutions in embodiments of this application.

In embodiments of this application, the wearable device may be divided into functional modules based on the foregoing method examples. For example, each functional module may be obtained through division based on each corresponding function, or two or more functions may be integrated into one processing unit. The integrated unit may be implemented in a form of hardware, or may be implemented in a form of software functional module. It should be noted that, in embodiments of this application, division into the units is an example, and is merely a logical function division. During actual implementation, another division manner may be used.

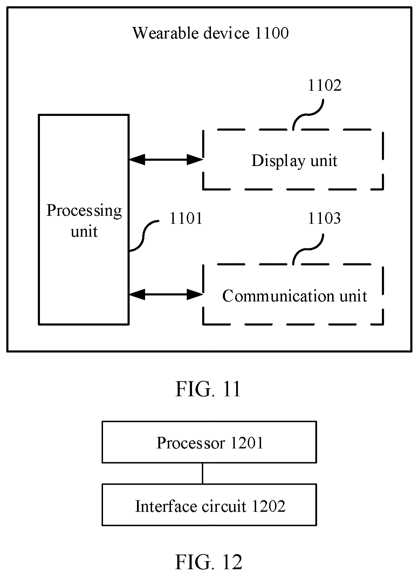

FIG. 11 is a diagram of a structure of a wearable device according to an embodiment of this application. The wearable device 1100 may be configured to implement the method performed by the wearable device described in the foregoing method embodiments. For example, the wearable device 1100 may specifically include a processing unit 1101.

The processing unit 1101 is configured to: start a first manner to measure user blood pressure in a first time period; determine a time period of abnormal blood pressure based on the user blood pressure and a measurement time period corresponding to the user blood pressure; and start a second manner to measure user blood pressure in the time period of abnormal blood pressure, where the second manner is different from the first manner.

In a possible design, the first manner includes a manner of determining user blood pressure based on at least one of a PGG signal, a pressure sensor signal, and an electrocardiogram. The second manner includes a manner of determining user blood pressure according to oscillography.

In a possible design, the processing unit 1101 is specifically configured to: if user blood pressure measured in a time period included in the first time period satisfies a preset blood pressure abnormality condition, determine that the time period is the time period of abnormal blood pressure.

In a possible design, the preset blood pressure abnormality condition includes at least one of a blood pressure condition, a fluctuation condition, and a quantity condition. The blood pressure condition includes: user blood pressure is not within a preset blood pressure range, and/or a user blood pressure curve does not match a preset curve. The fluctuation condition includes a first fluctuation condition, and the first fluctuation condition includes: a fluctuation value of user blood pressure is not within a preset fluctuation range. The quantity condition includes: for a time period, a proportion of a quantity of measured user blood pressure satisfying the blood pressure condition and/or the fluctuation condition to a quantity of time periods included in the first time period is within a first proportion range, or the quantity of measured user blood pressure satisfying the blood pressure condition and/or the fluctuation condition is within a first quantity range.

In a possible design, the time period of abnormal blood pressure is a daytime time period. The processing unit 1101 is specifically configured to start the second manner to measure the user blood pressure in the time period of abnormal blood pressure at high frequency.

In a possible design, the first time period includes n daytimes, and n is greater than 0. The processing unit 1101 is further configured to: if in the first time period, a quantity of daytimes among which a proportion of a total length of a time period of abnormal blood pressure included in a daytime to a total length of the daytime is within a second proportion range is greater than a second quantity range, or a quantity of daytimes among which a total length of a time period of abnormal blood pressure included in a daytime is within a first length range is greater than the second quantity range, start the second manner to measure user blood pressure in the daytimes.

In a possible design, the fluctuation condition further includes a second fluctuation condition and a third fluctuation condition. The second fluctuation condition includes: user blood pressure is greater than or equal to a first fluctuation threshold and is less than or equal to a second fluctuation threshold. The third fluctuation condition includes: user blood pressure is greater than the second fluctuation threshold.

In a possible design, the time period of abnormal blood pressure is at night. The processing unit 1101 is specifically configured to: if the second fluctuation condition is satisfied in the time period of abnormal blood pressure, start the second manner to measure the user blood pressure in the time period of abnormal blood pressure at low frequency.

In a possible design, the time period of abnormal blood pressure is at night. The processing unit 1101 is specifically configured to: if the third fluctuation condition is satisfied in the time period of abnormal blood pressure, start the second manner to measure the user blood pressure in the time period of abnormal blood pressure at high frequency.

In a possible design, the processing unit 1101 is further configured to output a reminder message, where the reminder message is used to remind a user to start, in the time period of abnormal blood pressure, the second manner to measure the user blood pressure.

In a possible design, the processing unit 1101 is further configured to determine that the time period of abnormal blood pressure is a daytime time period.

In a possible design, the processing unit 1101 is specifically configured to: receive a user operation; and in response to the user operation, start the second manner to measure the user blood pressure in the time period of abnormal blood pressure.

Optionally, the wearable device 1100 shown in FIG. 11 may further include a display unit 1102. The display unit 1102 is configured to support the wearable device 1100 in performing various display operations.

Optionally, the wearable device 1100 shown in FIG. 11 may further include a communication unit 1103. The communication unit 1103 is configured to support the wearable device 1100 in performing the step of communication between the wearable device and another device in embodiments of this application.

Optionally, the wearable device 1100 shown in FIG. 11 may further include a storage unit (not shown in FIG. 11), and the storage unit stores a program or instructions. When the processing unit 1101 executes the program or the instructions, the wearable device 1100 shown in FIG. 11 is enabled to perform the method shown in the foregoing method embodiments.

For technical effects of the wearable device 1100 shown in FIG. 11, refer to the technical effects of the method shown in the foregoing method embodiments. Details are not described herein again. The processing unit 1101 in the wearable device 1100 shown in FIG. 11 may be implemented by a processor or a processor-related circuit component, and may be a processor or a processing module. The communication unit 1103 may be implemented by a transceiver or a transceiver-related circuit component, and may be a transceiver or a transceiver module. The display unit 1102 may be implemented by a display-related component.

An embodiment of this application further provides a chip system. As shown in FIG. 12, the chip system includes at least one processor 1201 and at least one interface circuit 1202. The processor 1201 and the interface circuit 1202 may be connected to each other through a wire. For example, the interface circuit 1202 may be configured to receive a signal from another apparatus. For another example, the interface circuit 1202 may be configured to send a signal to another apparatus (for example, the processor 1201). For example, the interface circuit 1202 may read instructions stored in a memory, and send the instructions to the processor 1201. When the instructions are executed by the processor 1201, a wearable device is enabled to perform steps performed by the wearable device in the foregoing embodiments. Certainly, the chip system may further include another discrete component. This is not specifically limited in embodiments of this application.

Optionally, there may be one or more processors in the chip system. The processor may be implemented by using hardware, or may be implemented by using software. When the processor is implemented by using the hardware, the processor may be a logic circuit, an integrated circuit, or the like. When the processor is implemented by using the software, the processor may be a general-purpose processor, and is implemented by reading software code stored in a memory.

Optionally, there may also be one or more memories in the chip system. The memory may be integrated with the processor, or may be disposed separately from the processor. This is not limited in this application. For example, the memory may be a non-transitory processor, for example, a read-only memory ROM. The memory and the processor may be integrated into a same chip, or may be separately disposed on different chips. A type of the memory and a manner of disposing the memory and the processor are not specifically limited in this application.

For example, the chip system may be a field programmable gate array (field programmable gate array, FPGA), an application-specific integrated circuit (application-specific integrated circuit, ASIC), a system on chip (system on chip, SoC), a central processing unit (central processing unit, CPU), a network processor (network processor, NP), a digital signal processor (digital signal processor, DSP), a micro controller unit (micro controller unit, MCU), a programmable logic device (programmable logic device, PLD), or another integrated chip.

It should be understood that the steps in the foregoing method embodiments may be completed by using an integrated logic circuit of hardware in the processor or instructions in a form of software. The steps of the method disclosed with reference to embodiments of this application may be directly performed and completed by a hardware processor, or may be performed and completed through a combination of hardware in the processor and a software module.

An embodiment of this application further provides a computer storage medium. The computer storage medium stores computer instructions. When the computer instructions are run on a wearable device, the wearable device is enabled to perform the method in the foregoing method embodiments.

An embodiment of this application provides a computer program product. The computer program product includes a computer program or instructions. When the computer program or the instructions are run on a computer, the computer is enabled to perform the method in the foregoing method embodiments.

In addition, an embodiment of this application further provides an apparatus. The apparatus may be specifically a chip, a component, or a module. The apparatus may include a processor and a memory that are connected to each other. The memory is configured to store computer-executable instructions. When the apparatus runs, the processor may execute the computer-executable instructions stored in the memory, to enable the apparatus to perform the method in the foregoing method embodiments.

The wearable device, the computer storage medium, the computer program product, or the chip provided in embodiments is configured to perform the corresponding method provided above. Therefore, for beneficial effects that can be achieved by the wearable device, the computer storage medium, the computer program product, or the chip, refer to the beneficial effects in the corresponding method provided above. Details are not described herein again.

Based on the descriptions of the foregoing implementations, a person skilled in the art may understand that, for a purpose of convenient and brief description, division into the foregoing functional modules is used as an example for illustration. During actual application, the foregoing functions may be allocated to different functional modules and implemented according to requirements. In other words, an inner structure of an apparatus is divided into different functional modules to implement all or some of the foregoing functions.

In the several embodiments provided in this application, it should be understood that the disclosed apparatus and method may be implemented in other manners. Embodiments may be combined or referenced with each other without conflict. The described apparatus embodiments are merely examples. For example, the division into modules or units is merely logical function division and may be other division during actual implementation. For example, a plurality of units or components may be combined or integrated into another apparatus, or some features may be ignored or not performed. In addition, the displayed or discussed mutual couplings or direct couplings or communication connections may be implemented through some interfaces. The indirect couplings or communication connections between the apparatuses or units may be implemented in electrical, mechanical, or other forms.

The units described as separate parts may or may not be physically separate, and parts displayed as units may be one or more physical units, may be located in one place, or may be distributed to different places. Some or all of the units may be selected according to actual requirements to achieve the objectives of the solutions in embodiments.

In addition, functional units in embodiments of this application may be integrated into one processing unit, each of the units may exist alone physically, or two or more units may be integrated into one unit. The integrated unit may be implemented in a form of hardware, or may be implemented in a form of software functional unit.

When the integrated unit is implemented in the form of software functional unit and sold or used as an independent product, the integrated unit may be stored in a readable storage medium. Based on such an understanding, the technical solutions in embodiments of this application essentially, or the part contributing to the prior art, or all or some of the technical solutions may be implemented in a form of software product. The software product is stored in a storage medium and includes several instructions for instructing a device (which may be a single-chip microcomputer, a chip, or the like) or a processor (processor) to perform all or some of the steps of the method in embodiments of this application. The storage medium includes various media that can store program code, for example, a USB flash drive, a removable hard disk drive, a read-only memory (read-only memory, ROM), a random access memory (random access memory, RAM), a magnetic disk, or an optical disc.

The foregoing content is merely specific implementations of this application, but is not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

## Claims

1. A blood pressure measurement method, applied to a wearable device, wherein the method comprises:
starting a first manner to measure user blood pressure in a first time period;
determining a time period of abnormal blood pressure based on the user blood pressure and a measurement time period corresponding to the user blood pressure; and
starting a second manner to measure user blood pressure in the time period of abnormal blood pressure, wherein the second manner is different from the first manner.

2. The method according to claim 1, wherein
the first manner comprises a manner of determining user blood pressure based on at least one of a photoplethysmography PGG signal, a pressure sensor signal, and an electrocardiogram; and
the second manner comprises a manner of determining user blood pressure according to oscillography.

3. The method according to claim 1 or 2, wherein determining the time period of abnormal blood pressure based on the user blood pressure and the measurement time period corresponding to the user blood pressure comprises:
if user blood pressure measured in a time period comprised in the first time period satisfies a preset blood pressure abnormality condition, determining that the time period is the time period of abnormal blood pressure.

4. The method according to claim 3, wherein the preset blood pressure abnormality condition comprises at least one of a blood pressure condition, a fluctuation condition, and a quantity condition;
the blood pressure condition comprises: user blood pressure is not within a preset blood pressure range, and/or a user blood pressure curve does not match a preset curve;
the fluctuation condition comprises a first fluctuation condition, and the first fluctuation condition comprises: a fluctuation value of user blood pressure is not within a preset fluctuation range; and
the quantity condition comprises: for a time period, a proportion of a quantity of measured user blood pressure satisfying the blood pressure condition and/or the fluctuation condition to a quantity of time periods comprised in the first time period is within a first proportion range, or the quantity of measured user blood pressure satisfying the blood pressure condition and/or the fluctuation condition is within a first quantity range.

5. The method according to any one of claims 1 to 4, wherein the time period of abnormal blood pressure is a daytime time period; and
starting the second manner to measure the user blood pressure in the time period of abnormal blood pressure comprises:
starting the second manner to measure the user blood pressure in the time period of abnormal blood pressure at high frequency.

6. The method according to any one of claims 1 to 5, wherein the first time period comprises n daytimes, and n is greater than 0; and
after determining the time period of abnormal blood pressure based on the user blood pressure and the measurement time period corresponding to the user blood pressure, the method further comprises:
if in the first time period, a quantity of daytimes among which a proportion of a total length of a time period of abnormal blood pressure comprised in a daytime to a total length of the daytime is within a second proportion range is greater than a second quantity range, or a quantity of daytimes among which a total length of a time period of abnormal blood pressure comprised in a daytime is within a first length range is greater than the second quantity range, starting the second manner to measure user blood pressure in the daytimes.

7. The method according to claim 4, wherein the fluctuation condition further comprises a second fluctuation condition and a third fluctuation condition;
the second fluctuation condition comprises: user blood pressure is greater than or equal to a first fluctuation threshold and is less than or equal to a second fluctuation threshold; and
the third fluctuation condition comprises: user blood pressure is greater than the second fluctuation threshold.

8. The method according to claim 7, wherein the time period of abnormal blood pressure is at night; and
starting the second manner to measure the user blood pressure in the time period of abnormal blood pressure comprises:
if the second fluctuation condition is satisfied in the time period of abnormal blood pressure, starting the second manner to measure the user blood pressure in the time period of abnormal blood pressure at low frequency.

9. The method according to claim 7, wherein the time period of abnormal blood pressure is at night; and
starting the second manner to measure the user blood pressure in the time period of abnormal blood pressure comprises:
if the third fluctuation condition is satisfied in the time period of abnormal blood pressure, starting the second manner to measure the user blood pressure in the time period of abnormal blood pressure at high frequency.

10. The method according to any one of claims 1 to 9, wherein before starting the second manner to measure the user blood pressure in the time period of abnormal blood pressure, the method further comprises:
outputting a reminder message, wherein the reminder message is used to remind a user to start, in the time period of abnormal blood pressure, the second manner to measure the user blood pressure.

11. The method according to claim 10, wherein before outputting the reminder message, the method further comprises:
determining that the time period of abnormal blood pressure is a daytime time period.

12. The method according to any one of claims 1 to 11, wherein starting the second manner to measure the user blood pressure in the time period of abnormal blood pressure comprises:
receiving a user operation; and
in response to the user operation, starting the second manner to measure the user blood pressure in the time period of abnormal blood pressure.

13. A wearable device, comprising a processor, a memory, and a sensor, wherein the memory and the sensor are coupled to the processor, the memory is configured to store computer program code, the computer program code comprises computer instructions, and the processor reads the computer instructions from the memory, to enable the wearable device to perform the method according to any one of claims 1 to 12.

14. A computer-readable storage medium, wherein the computer-readable storage medium comprises a computer program or instructions, and when the computer program or the instructions are run on a wearable device, the wearable device is enabled to perform the method according to any one of claims 1 to 12.

15. A computer program product, wherein when the computer program product is run on a computer, the computer is enabled to perform the method according to any one of claims 1 to 12.
